# EUROPEAN PATENT APPLICATION

(11) **EP 4 538 392 A1**
(43) Date of publication of application: **16.04.2025**
(21) Application number: 23383045.4
(22) Date of filing: 11.10.2023
(51) Int. Cl.: C12Q 1/6886

(54) **GENETIC SIGNATURE OF LUNG CANCER**

(71) Applicant: Pangaea Oncology, S.A, 08028 Barcelona (ES)
(72) Inventor: PEDRAZ VALDUNCIEL, Carlos, E-08028 Barcelona (ES); GIMÉNEZ CAPITÁN, Ana María, E-08028 Barcelona (ES)
(74) Representative: ABG Intellectual Property Law, S.L.

(57) **Abstract**

The present invention relates to a method which allows for determining if the lung nodules of a patient are benign or cancerous. Furthermore the method also allows to determine if a patient is at high risk of developing lung cancer and if it should be subject to medical intervention.

## Description

### TECHNICAL FIELD OF THE INVENTION

The present invention relates to the field of medicine, more in particular, to methods to diagnose and predict the evolution of lung cancer in a subject.

### BACKGROUND OF THE INVENTION

Cancer caused an estimated 10 million deaths worldwide in 2020. This high mortality rate is largely a consequence of the lack of early detection tools for many tumors, which means that most are diagnosed in non-surgical stages, with a significantly worse prognosis. Although new therapies have been developed for some neoplasms in recent years, survival rates in advanced stages remain low. In the case of non-small cell lung cancer (NSCLC), 75-85% of cases are diagnosed in metastatic stages IIIB-IV, which have a 5-year survival rate of 17-20%. In contrast, patients diagnosed in early stages (I-IIIA) can undergo surgery with significantly better survival rates of 80-90% at 5 years.

Diagnosis of early-stage lung cancer is rare and often discovered incidentally when studying other diseases, because the symptoms in the early stages are very nonspecific. These cases may be asymptomatic, leading to a delay in diagnosis. Imaging technologies often detect pulmonary nodules of unknown significance, and as a result, some patients with benign nodules undergo unnecessary surgery, while others with small tumors are kept under observation, depriving them of potentially beneficial early treatment.

Both primary tumors and metastases, if they exist, release materials into the blood and other fluids (pleural, ascitic, cerebrospinal fluids, etc.), which can be used as "liquid biopsy". The information generated by "liquid" biopsies complements tissue biopsies, which are currently irreplaceable sources of information that cannot be obtained by any other means, such as tumor type and histology. Still, blood and other "liquid biopsies" have some advantages. Thus, it can be obtained repeatedly without risk and used to monitor the course of the disease, including early detection of response and relapse or the appearance of resistance to a particular therapy.

A gene expression signature would be particularly useful to guide the clinical decision in patients with pulmonary nodules suspected of malignancy, directing towards surgery or observation.

### SUMMARY OF THE INVENTION

The inventors have determined a gene signature which allows to differentiate between benign and cancerous lung nodules present in a patient with an improved accuracy.

Therefore, a first aspect of the present invention relates to a method for determining whether lung nodules in a patient are benign or cancerous comprising:
(i) determining the expression level of the MS4A1 gene in a sample from the subject, and
(ii) comparing the level obtained in (i) to a reference value,
wherein an expression level of the MS4A1 gene that is decreased with respect to the reference value is indicative that the lung nodules are cancerous nodules.

Another aspect of the present invention relates to a method for selecting a subject having one or more lung nodules for lung cancer therapy comprising:
(i) determining the expression level of the MS4A1 gene in a sample from the subject, and
(ii) comparing the level obtained in (i) to a reference value,
wherein an expression level of the MS4A1 gene that is decreased with respect to the reference value is indicative that the subject is selected for a lung cancer therapy

A further aspect of the present invention relates to a method for selecting a subject having one or more lung nodules for lung cancer therapy and treating the subject with said therapy comprising:
(i) determining the expression level of the MS4A1 gene in a sample from the subject, and
(ii) comparing the level obtained in (i) to a reference value,
(iii) treating the subject with the therapy if the subject is identified as having decreased expression level of the MS4A1 gene with respect to the reference value.

Yet another aspect of the present invention relates to a method for determining whether lung nodules in a patient are benign or are cancerous comprising:
(i) determining the level of the hsa_circ_0000994 and/or hsa_circ_0012673 circular RNAs in a sample from the patient, and
(ii) comparing the level obtained in (i) to a reference value,
wherein an increased level of the hsa_circ_0000994 circular RNA and/or a decreased level of the hsa_circ_0012673 circular RNA with respect to their respective reference values is indicative that the lung nodules are cancerous nodules.

A further aspect of the invention relates to a method for selecting a subject having one or more lung nodules for lung cancer therapy comprising:
(i) determining the level of the hsa_circ_0000994 and/or hsa_circ_0012673 circular RNAs in a sample from the patient, and
(ii) comparing the level obtained in (i) to a reference value,
wherein an increased level of the hsa_circ_0000994 circular RNA and/or a decreased level of the hsa_circ_0012673 circular RNA with respect to their respective reference values is indicative that the subject is selected for lung cancer therapy.

Yet another aspect of the present invention relates to a method for selecting a subject having one or more lung nodules for lung cancer therapy and treating the subject with said therapy comprising:
(i) determining the level of the hsa_circ_0000994 and/or hsa_circ_0012673 circular RNAs in a sample from the patient, and
(ii) comparing the level obtained in (i) to a reference value,
(iii) treating the subject with the therapy if the patient is identified as having increased levels of the hsa_circ_0000994 circular RNA and/or a decreased level of the hsa_circ_0012673 circular RNA with respect to their respective reference values.

Another aspect of the present invention relates to a kit comprising specific reagents for carrying out the method according to the invention.

A further aspect of the present invention relates to a computer system comprising one or more programs, wherein the one or more programs are include instructions for performing the method according to the invention.

A final aspect of the present invention relates to a computer-readable storage medium storing one or more programs, the one or more programs comprising instructions, which when executed by one or more processors of an electronic device, cause the electronic device to perform the method according to the invention.

### BRIEF DESCRIPTION OF THE FIGURES

**Figure 1****.** Volcano plot representing the Log2(FC) and nominal -Log10(p-values) of all transcripts included in the 10360 panel for Cancer vs. healthy and healthy donor. Total of 750 features.

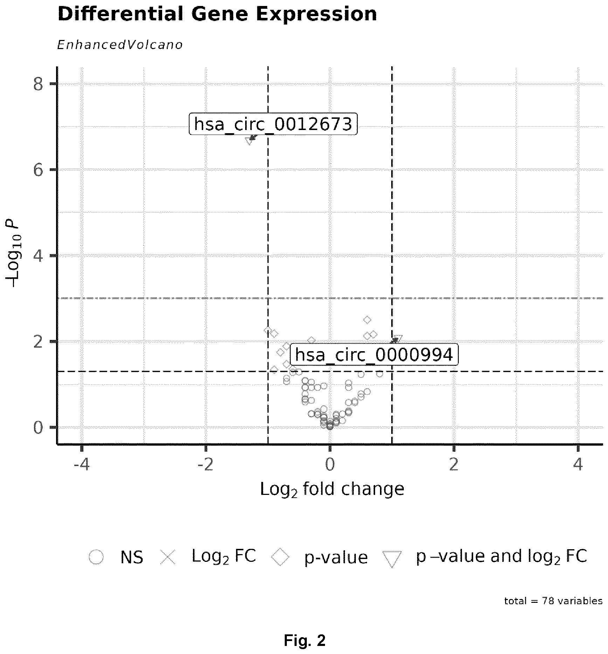

**Figure 2****.** Volcano plot representing the Log2(FC) and nominal -Log10(p-values) of all transcripts included in a nCounter custom-made panel for Cancer vs. healthy and healthy donor. Total of 78 features.

### DETAILED DESCRIPTION OF THE INVENTION

The inventors have identified transcriptomic signatures based on the levels of different mRNA and circRNA in biofluids that allow the determination in a patient showing one or more lung nodules whether the nodule is benign or cancerous.

### Method for determining cancerous lung nodules

In a first aspect of the present invention relates to a method (hereinafter "Diagnostic method of the invention) for determining whether lung nodules in a patient are benign or are cancerous comprising:
(i) determining the expression level of MS4A1 in a sample from the subject, and
(ii) comparing the level obtained in (i) to a reference value,
wherein an expression level of MS4A1 that is decreased with respect to the reference value is indicative that the lung nodules are cancerous nodules.

The diagnostic method of the invention can also be carried out by:
(i) determining the level of the hsa_circ_0000994 and/or hsa_circ_0012673 circular RNAs in a sample from the patient, and
(ii) comparing the level obtained in (i) to a reference value,
wherein an increased level of the hsa_circ_0000994 circular RNA and/or a decreased level of the hsa_circ_0012673 circular RNA with respect to their respective reference values is indicative that the lung nodules are cancerous nodules.

The term "lung nodule" as used herein is interchangeable with "pulmonary lesion". A lung nodule refers to abnormal growths, small lumps, swelling or collection of tissue in the lung of a patient. Around 95% of lung nodules are benign and are due to previous infections or caring of the lung tissue. Despite this, in 5% of the cases the lung nodules are cancerous. The skilled person will be aware of common techniques which are used to detect lung nodules such as X-Ray and/or chest computerized tomography scans (CT scans). While lung nodules detected by X-Ray are usually larger than 0.5 cm, CT scans can detect lung nodules which are smaller than 6 mm. In a particular embodiment of the method I of the invention the lung nodules are detected by chest X-ray and/or chest CT scan. A pulmonary nodule is any nodule less than or equal to three centimeters in diameter. In some embodiments, a pulmonary nodule has a diameter of about 0.8 cm to 2 cm.

The present diagnostic method of the invention permits the detection of lung nodules which are cancerous nodules. The term "cancerous nodules" as used herein refers to those nodules affected by or showing abnormalities characteristic of lung cancer.

The term "lung cancer", as used herein, refers to a neoplasm, e.g., a malignant neoplasm, of the lung of the patient, wherein the neoplasm is of epithelial origin (i.e., carcinoma of the lung). Lung carcinomas are categorized by the size and appearance of the malignant cells and the term "lung cancer" includes both non-small cell lung cancer (NSCLC) and small cell lung cancer (SCLC). NSCLC is any type of epithelial lung cancer other than SCLC. The most common types of NSCLC are squamous cell carcinoma, large cell carcinoma, and adenocarcinoma, but there are several other types that occur less frequently, and all types can occur in unusual histological variants. In some embodiments, the diagnostic method of the invention allows the determination of the tumor TNM stage (e.g., stage IA, stage IB, stage IIA, and/or the like), tumor grade (e.g., well-differentiated, moderate, poorly-differentiated, undifferentiated, and/or the like), a tumor subtype (e.g., adenocarcinoma, squamous cell carcinoma, small cell carcinoma, and/or the like), a tumor histology (e.g., invasive carcinoma, metastasis, carcinoma in situ, and/or the like), and/or the like.

In a particular embodiment of the diagnostic method of the invention, the method allows the identification of the nodules as nodules from non-small cell lung cancer (NSCLC).

The term "patient" or "subject" refers to all animals classified as mammals and includes but is not limited to domestic and farm animals, primates and humans, for example, human beings, non-human primates, cows, horses, pigs, sheep, goats, dogs, cats, or rodents. Preferably, the subject is a human man or woman of any age or race. More preferably, the subject is a human adult, i.e having 18 years or more.

In some embodiments, the patient has been previously identified as carrying lung nodules by imaging techniques. As used herein, "imaging" may refer to an image (e.g., an X-ray, a CT scan, and/or the like) obtained by one or more radiography techniques, such as X-ray imaging, CT imaging (e.g., non-contrast CT imaging, contrast CT imaging, and/or the like), magnetic resonance imaging (MRI), positron-emission tomography (PET) imaging, and/or the like. In some implementations, the imaging described herein may be chest imaging that depicts an area below a neck of a patient and above a bowel of the patient. In such a case, the chest imaging may depict a cardiovascular system and/or a pulmonary system of the patient. For example, the chest imaging may depict lungs of the patient.

### Step (i) of the diagnostic method

The first step of the diagnostic method of the invention relates to the determination of the expression levels of the MS4A1 gene or to the determination of the levels of the hsa_circ_0000994 and/or the hsa_circ_0012673 circular RNAs in a sample from the patient.

The expression "determination of the expression levels of the MS4A1 gene" refers to the determination of any value which provides an indication on the overall expression the gene, which includes either the mRNA levels or the levels of the protein encoded by the MS4A1 gene, when such expression is measured quantitatively, based on methods as disclosed herein and/or routinely known to the skilled person. The term "expression level", as used herein, refers to a measurable quantity of a gene product produced by the gene in a sample of the subject, wherein the gene product can be a transcriptional product or a translational product. As understood by the person skilled in the art, the gene expression level can be quantified by measuring the levels of the transcriptional product of said gene (messenger RNA when the gene is a protein-coding gene) or of the protein encoded by said gene. In a particular embodiment of the method I of the invention the determination of the expression level of the gene is carried out by determining the levels of RNA transcripts of the gene(s).

The level of a given RNA transcript can be determined by methods well known in the art. For example the nucleic acid contained in the sample is first extracted according to standard methods, for example using lytic enzymes or chemical solutions or extracted by nucleic-acid-binding resins following the manufacturer's instructions. The extracted RNA is then detected by hybridization (e.g., Northern blot analysis, y oligonucleotide microarrays after converting the mRNA into a labeled cDNA or by methods based on the binding of specific probes labelled with colour-coded molecular barcodes to the biomarker of interest sucha s nCounter.) and/or amplification (e.g., RT-PCR). Quantitative or semi-quantitative RT-PCR is preferred. Real-time quantitative or semiquantitative RT-PCR is particularly advantageous. Preferably, primer pairs are designed in order to overlap an intron, so as to distinguish cDNA amplification from putative genomic contamination. Suitable primers may be easily designed by the skilled person. Other methods of amplification include ligase chain reaction (LCR), transcription-mediated amplification (TMA), strand displacement amplification (SDA) and nucleic acid sequence based amplification (NASBA).

Alternatively the first step of the diagnostic method of the invention comprises the determination of the levels of the hsa_circ_0000994 and/or hsa_circ_0012673 circular RNAs in the sample.

As used herein, the terms "circular RNA" or "circular polyribonucleotide" or "circRNA" or "oRNA" are used interchangeably and refers to a polyribonucleotide that forms a circular structure through covalent bonds. Preferably, the circular RNA or RNAs to be used in the methods of the present invention is single stranded, thereby enabling translation of the RNA.

Methods for the detection or nucleic acid molecules such as circRNA in a sample comprise PCR based methods which involve the target specific amplification of nucleic acids. However, any nucleic acid detection assay known to the skilled artisan may be used to detect one or more circRNA of the invention. In a preferred embodiment, the level of circRNA transcripts can be determined by using the rolling circle amplification. As used herein, the term "nucleic acid detection assay" refers to any method of determining the nucleotide composition of a nucleic acid of interest. Nucleic acid detection assay include but are not limited to, sequencing methods, probe hybridization methods, structure specific cleavage assays (e.g., the INVADER assay, (Hologic, Inc); enzyme mismatch cleavage methods; polymerase chain reaction (PCR); branched hybridization methods; rolling circle replication; NASBA; molecular beacon technology; E-sensor technology (Motorola, U.S. Pat. No. 6,248,229); cycling probe technology; Dade Behring signal amplification methods; ligase chain reaction; and sandwich hybridization methods.

The methods of the invention may preferably include a further step of removing ribosomal and/or poly A RNA from the biological sample before determining the level of a circRNA of the invention. Thus, before determining the level of the circRNA of the invention, the biological sample or the isolated RNA therefrom is treated to remove any ribosomal and/or poly-A tail RNA (mRNA). Methods to remove rRNA or polyA RNA are well known in the art and are exemplary described in the example section of the present application. By removing any non circRNA from the sample, the results are more specific and sensitive due to a reduced RNA background "noise". This step may be performed in any aspect or embodiment of the invention which requires the detection or isolation of a circRNA of the invention.

In a particular embodiment of the diagnostic method of the invention of the invention, the determination of the levels of the RNA transcripts and/or circular RNA is carried out by measuring the levels of circulating RNA. The term "circulating RNA" is used interchangeably with "circulating free "circulating cell-free RNA" and refers to RNA (mRNA or circRNA) which is found free from cells, primarily in the blood, plasma or serum in circulation in the subject's body. Circulating free RNA is normally found encapsulated within extracellular membrane vesicles or they form ribonucleoprotein complexes.

MS4A1 protein refers to a B-lymphocyte-specific membrane protein that plays a role in the regulation of cellular calcium influx necessary for the development, differentiation, and activation of B-lymphocytes and is defined in the Ensembl database with accession number ENSG00000173334.4, release 109 of February 2023. In some embodiments, the methods of the invention comprises the determination of the levels of one or more of the ENST00000345732.9, the ENST00000534668.6, the ENST00000389939.2, the ENST00000532073.5. the ENST00000674194.1, the ENST00000528313.1, the ENST00000533306.6, the ENST00000532491.5, the ENST00000534503.5, the ENST00000527101.5, the ENST00000532418.1 or the ENST00000530482.1 transcripts (accession numbers for the transcripts as provided in the Ensemble database release 110 of July 2023).

The first step of the diagnostic method of the invention based on the determination of circRNA levels comprises the determination of the expression level of the hsa_circ_0000994 and/or of the hsa_circ_0012673 circular RNAs, wherein the circRNAs are as defined in the circBase database under accession numbers hsa_circ_0000994 an hsa_circ_0012673 respectively. In a particular embodiment hsa_circ_0012673 refers to the sequence according to SEQ ID NO: 2. In a particular embodiment hsa_circ_0012673 refers to the sequence according to SEQ ID NO: 3.

It will be understood that the determination of the levels of the MS4A1 transcript or of the levels of the hsa_circ_0000994 and/or of the hsa_circ_0012673 can be carried out by measuring the levels of a fragment of each of these transcripts. The term "fragment thereof" as used herein refers to a short sequence of RNA which allows the unambiguous identification of the gene in question. In a particular embodiment the fragment has a sequence length of between 100 and the full length minus 1 nucleotide of the polynucleotides defined above as SEQ ID NO: 1 to 3.

In the present invention, the term "sample" or "biological sample" means biological material isolated from a subject. The biological sample can contain any biological material suitable for detecting the expression levels of the desire genes, that is, the sample should contain mRNA and/or proteins or the levels of circRNA, preferably mRNA and/or proteins or circRNA from the lung nodules, and can comprise cell and/or non-cell material of the subject. In a particular embodiment, the sample is a tumor sample. In a particular embodiment of the method I of invention the sample is selected form blood, plasma or serum. In a more particular embodiment of the method I of the invention the sample is plasma.

In order to improve the accuracy of the diagnostic method of the invention based on the determination of the expression levels of the MS4A gene, the expression levels of further genes can be determined in step (i) of the method. In a particular embodiment of diagnostic method of the invention, step (i) further comprises determining the expression level of DDB2 in the sample from the patient, wherein an expression level of DDB2 that is increased with respect to the reference value is indicative that the lung nodules are cancerous nodules.

The term "DDB2" refers to the gene which encodes for the protein DNA damage-binding protein 2 which is involved in DNA repair and protein ubiquitination as part of the UV-DDB complex and as part of the DCX (DDB1-CUL4-X-box) complex. The expression levels DDB2 gene can be determined by the levels of the corresponding mRNA or by the levels of the polypeptide encoded by said gene. In a preferred embodiment, the expression levels of the DDB2 gene is determined by determining the expression levels of one or more of the transcripts defined under the accession numbers ENST00000256996.9, ENST00000378603.7, ENST00000614825.4, ENST00000378600.7, ENST00000622090.4, ENST00000622878.4, ENST00000617847.4, ENST00000378601.7, ENST00000616278.4, ENST00000610805.4, ENST00000620515.1, ENST00000612309.4, ENST00000617022.4, ENST00000614394.1, ENST00000615695.1 and ENST00000614884.1 in the Ensemble database under accession number ENSG00000134574 dated (Release 110). In a preferred embodiment, the expression levels of the DDB2 gene are determined by measuring the expression of the polypeptide defined under accession number Q92466 of the Uniprot database, entry version 28-Jun-2023..

In a further particular embodiment of diagnostic method of the invention step (i) further comprises determining the expression level of at least one gene selected from group consisting of: SPIB, TAPBPL, TLR8, LY9, RELA, CXCL8, CD74, KLRK1, CD3D, DUSP1, wherein the alteration of the expression level of said at least one gene with respect to the reference value is indicative that the lung nodules are cancerous nodules.

The term "SPIB" refers to the gene which encodes for the protein "Transcription factor Spi-B" which is a sequence specific transcriptional activator which binds to the PU-boxthat can act as a lymphoid-specific enhancer. The expression levels SPIB gene can be determined by the levels of the corresponding mRNA or by the levels of the polypeptide encoded by said gene. In a preferred embodiment, the expression levels of the SPIB gene is determined by determining the expression levels of one or more of the transcripts defined under the accession numbers ENST00000595883.6, ENST00000439922.6, ENST00000270632.7, ENST00000596074.5, ENST00000597855.5, ENST00000594685.1, ENST00000599923.5 and ENST00000594188.1 in the Ensemble database under accession number ENSG00000269404 dated (Release 110). In a preferred embodiment, the expression levels of the DDB2 gene are determined by measuring the expression of the polypeptide defined under accession number Q01892 of the Uniprot database, entry version 28-Jun-2023..

The term "TAPBPL" refers to the gene which encodes for the protein "Tapasin-related protein" which is a component of the antigen processing and presentation pathway, which binds to MHC class I coupled with beta2-microglobulin/B2M. The expression levels TAPBL gene can be determined by the levels of the corresponding mRNA or by the levels of the polypeptide encoded by said gene. In a preferred embodiment, the expression levels of the TAPBL gene is determined by determining the expression levels of one or more of the transcripts defined under the accession numbers ENST00000266556.8, ENST00000544021.5, ENST00000545700.5, ENST00000539384.5, ENST00000543567.5, ENST00000544289.1, ENST00000544826.5 and ENST00000542160.1 in the Ensemble database under accession number ENSG00000139192 dated (Release 110).. In a preferred embodiment, the expression levels of the TAPBLP gene are determined by measuring the expression of the polypeptide defined under accession number Q9BX59 of the Uniprot database, entry version 28-Jun-2023.

The term "TLR8" refers to the gene which encodes for the protein "Toll-like receptor 8". The expression levels TLR8 gene can be determined by the levels of the corresponding mRNA or by the levels of the polypeptide encoded by said gene. In a preferred embodiment, the expression levels of the TLR8 gene is determined by determining the expression levels of one or more of the transcripts defined under the accession numbers ENST00000218032.7 and ENST00000311912.5 in the Ensemble database under accession number ENSG00000101916dated (Release 110).. In a preferred embodiment, the expression levels of the TLR8 gene are determined by measuring the expression of the polypeptide defined under accession number Q9NR97 of the Uniprot database, entry version 28-Jun-2023.

The term "LY9" refers to the gene which encodes for the protein "T-lymphocyte surface antigen Ly-9" which is part of the Self-ligand receptor of the signaling lymphocytic activation molecule (SLAM) family. The expression levels LY9 gene can be determined by the levels of the corresponding mRNA or by the levels of the polypeptide encoded by said gene. In a preferred embodiment, the expression levels of the LY9 gene is determined by determining the expression levels of one or more of the transcripts defined under the accession numbers ENST00000263285.11, ENST00000368037.9, ENST00000392203.8, ENST00000368035.1, ENST00000368039.2, ENST00000474998.1, ENST00000490902.1, ENST00000471816.1, ENST00000485624.1, ENST00000480837.5 and ENST00000479663.2 in the Ensemble database under accession number ENSG00000122224 dated (Release 110).. In a preferred embodiment, the expression levels of the LY9 gene are determined by measuring the expression of the polypeptide defined under accession number Q9HBG7 of the Uniprot database, entry version 28-Jun-2023.

The term "RELA" refers to the gene which encodes for the protein "Transcription factor p65" which is a REL-associated protein involved in NF-κB heterodimer formation, nuclear translocation and activation. The expression levels RELA gene can be determined by the levels of the corresponding mRNA or by the levels of the polypeptide encoded by said gene. In a preferred embodiment, the expression levels of the RELA gene is determined by determining the expression levels of one or more of the transcripts defined under the accession numbers ENST00000406246.8, ENST00000525693.5, ENST00000308639.13, ENST00000612991.4, ENST00000532999.5, ENST00000527749.5, ENST00000534558.5, ENST00000527874.1, ENST00000534283.1, ENST00000532879.5, ENST00000533187.5, ENST00000526257.1, ENST00000526283.6, ENST00000529389.5, ENST00000525658.5, ENST00000533546.5, ENST00000531238.1, ENST00000527909.5, ENST00000531484.5, ENST00000526738.1, ENST00000525858.5, ENST00000534305.1, ENST00000525301.5, ENST00000532776.5, ENST00000527074.5 and ENST00000529330.1 in the Ensemble database under accession number ENSG00000173039 dated (Release 110).. In a preferred embodiment, the expression levels of the RELA gene are determined by measuring the expression of the polypeptide defined under accession number Q9HBG7 of the Uniprot database, entry version 28-Jun-2023.

The term "CXCL8" refers to the gene which encodes for the protein "Interleukin-8" which is a chemotactic factor that mediates inflammatory response by attracting neutrophils, basophils, and T-cells to clear pathogens and protect the host from infection; also plays an important role in neutrophil activation.. The expression levels CXCL8 gene can be determined by the levels of the corresponding mRNA or by the levels of the polypeptide encoded by said gene. In a preferred embodiment, the expression levels of the CXCL8 gene is determined by determining the expression levels of one or more of the transcripts defined under the accession numbers ENST00000307407.8, ENST00000401931.1, ENST00000696131.1, ENST00000696132.1 and ENST00000483500.1 in the Ensemble database under accession number ENSG00000169429 dated (Release 110).In a preferred embodiment, the expression levels of the CXCL8 gene are determined by measuring the expression of the polypeptide defined under accession number P10145 of the Uniprot database, entry version 28-Jun-2023.

The term "CD74" refers to the gene which encodes for the protein "HLA class II histocompatibility antigen gamma chain" which plays a critical role in MHC class II antigen processing by stabilizing peptide-free class II alpha/beta heterodimers in a complex soon after their synthesis and directing transport of the complex from the endoplasmic reticulum to the endosomal/lysosomal system where the antigen processing and binding of antigenic peptides to MHC class II takes place. The expression levels CD74 gene can be determined by the levels of the corresponding mRNA or by the levels of the polypeptide encoded by said gene. In a preferred embodiment, the expression levels of the CD74 gene is determined by determining the expression levels of one or more of the transcripts defined under the accession numbers ENST00000009530.13, ENST00000353334.11, ENST00000377795.7, ENST00000518797.6, ENST00000523208.6, ENST00000522246.5, ENST00000524315.6, ENST00000523813.2, ENST00000523836.6, ENST00000517791.1, ENST00000517752.5 and ENST00000522153.1 in the Ensemble database under accession number ENSG00000019582 dated (Release 110).. In a preferred embodiment, the expression levels of the CD74 gene are determined by measuring the expression of the polypeptide defined under accession number P10145 of the Uniprot database, entry version 28-Jun-2023.

The term "KLRK1" refers to the gene which encodes for the protein "NKG2-D type II integral membrane protein" which functions as an activating and costimulatory receptor involved in immunosurveillance upon binding to various cellular stress-inducible ligands displayed at the surface of autologous tumor cells and virus-infected cells. The expression levels KLRK1 gene can be determined by the levels of the corresponding mRNA or by the levels of the polypeptide encoded by said gene. In a preferred embodiment, the expression levels of the KLRK1 gene is determined by determining the expression levels of one or more of the transcripts defined under the accession numbers ENST00000240618.11, ENST00000540818.5, ENST00000396451.4, ENST00000544449.5 and ENST00000540267.5 in the Ensemble database under accession number ENSG00000213809 dated (Release 110).. In a preferred embodiment, the expression levels of the KLRK1 gene are determined by measuring the expression of the polypeptide defined under accession number P26718 of the Uniprot database, entry version 28-Jun-2023.

The term "CD3D" refers to the gene which encodes for the protein "T-cell surface glycoprotein CD3 delta chain". The expression levels CD3D gene can be determined by the levels of the corresponding mRNA or by the levels of the polypeptide encoded by said gene. In a preferred embodiment, the expression levels of the CD3D gene is determined by determining the expression levels of one or more of the transcripts defined under the accession numbers ENST00000300692.9, ENST00000392884.2, ENST00000529594.5, ENST00000534687.5, ENST00000695668.1, ENST00000695666.1, ENST00000695667.1 and ENST00000526561.1 in the Ensemble database under accession number ENSG00000213809 dated (Release 110).. In a preferred embodiment, the expression levels of the CD3D gene are determined by measuring the expression of the polypeptide defined under accession number P04234 of the Uniprot database, entry version 28-Jun-2023.

The term "DUSP1" refers to the gene which encodes for the protein "Dual specificity protein phosphatase 1" which dephosphorylates MAP kinase MAPK1/ERK2 on both 'Thr-183' and 'Tyr-185'. The expression levels DUSP1 gene can be determined by the levels of the corresponding mRNA or by the levels of the polypeptide encoded by said gene. In a preferred embodiment, the expression levels of the DUSP1 gene is determined by determining the expression levels of the transcript defined under the accession numbers ENST00000239223.4 in the Ensemble database under accession number ENSG00000120129 (Release 110).. In a preferred embodiment, the expression levels of the DUSP1 gene are determined by measuring the expression of the polypeptide defined under accession number P28562 of the Uniprot database, entry version 28-Jun-2023.

### Step (ii) of the method of the invention

The second step of the invention relates to the comparison of the expression level of the gene or the expression level of the circRNA obtained in step (i) of the method and comparing it to a reference value.

The term "reference value", as used herein, relates to a predetermined criteria used as a reference for evaluating the values or data obtained from the samples collected from a subject. The reference value or reference level can be an absolute value, a relative value, a value that has an upper or a lower limit, a range of values, an average value, a median value, a mean value, or a value as compared to a particular control or baseline value. A reference value can be based on a large number of samples, such as from population of subjects of the chronological age matched group, or based on a pool of samples. In a particular embodiment of the method I of the invention the reference value relates to the expression level of the gene or genes in a patient or a group of patients with benign lung nodules. In a particular embodiment the group of patients if of at least 2, at least 100, at least 200, at least 300, at least 400, at least 500, at least 600, at least 700, at least 800, at least 900, at least 1000 patients. Typically, the reference value is the value of the same variable which is measured (the expression levels of a gene or combination of genes or the levels of a circRNA) determined in a control sample.

Such a control sample may comprise any suitable sample, including but not limited to a sample from a control lung cancer patient (can be stored sample or previous sample measurement) with a known outcome; normal tissue or cells isolated from a subject, such as a normal patient or the lung cancer patient, cultured primary cells/tissues isolated from a subject such as a normal subject or the lung cancer patient, adjacent normal cells/tissues obtained from the same organ or body location of the lung cancer patient, a tissue or cell sample isolated from a normal subject, or a primary cells/tissues obtained from a depository. In another preferred embodiment, the control may comprise a reference gene expression level or a circRNA levels from any suitable source, including but not limited to housekeeping genes, an antigen level range from normal tissue (or other previously analyzed control sample), a previously determined level range within a test sample from a group of patients, or a set of patients with a certain outcome or receiving a certain treatment. It will be understood by those of skill in the art that such reference levels obtained from control samples can be used in combination as controls in the methods of the present invention.

When the expression level of the gene of interest is determined as the levels of mRNA or protein or of the circRNA, then the reference value should also be obtained from mRNA, protein or circRNA levels. When the expression level of the gene of interest is determined as the level of the protein encoded by said gene, then the reference value should also be obtained from protein levels.

The expression levels of the genes in question and/or of the reference value can be normalized in order to remove variations due to other factors such as the age, weight, sex, general physical condition of the patient and the like. The term "normalization" or "normalizer" as used herein refers to the expression of a differential value in terms of a standard value to adjust for effects which arise from technical variation due to sample handling, sample preparation and measurement rather than biological variation of protein concentration in a sample. For example, when measuring the expression of a differentially expressed protein, the absolute value for the expression of the protein can be expressed in terms of an absolute value for the expression of a standard protein that is substantially constant in expression. This prevents the technical variation of sample preparation and measurement from impeding the measurement of protein concentration levels in the sample.

As the expert is aware a common source of genes used for normalization of expression levels are housekeeping genes. The term "housekeeping gene" in the present context refers to any gene(s), which display consistent expression under normal and patho-physiological conditions, such that their expression levels can be used to normalize gene expression in a biological sample. In a particular embodiment of the method I of the invention the expression levels are normalized based on the expression level of at least one housekeeping gene. In a more particular embodiment of the diagnostic method of the invention based on the determination of the expression levels of one or more genes, the expression levels are normalized using the expression levels of one or more housekeeping genes selected from a group consisting of: OAZ1, UBB, MRPL19 and any combination thereof. In a more particular embodiment of the diagnostic method of the invention based on the determination of the levels of circRNAs, the levels are normalized using the expression levels of one or more housekeeping genes selected from a group consisting of the UBB, the MRPL19 genes or a combination thereof.

The term "OAZ1" refers to the gene which encodes for the protein "Ornithine decarboxylase antizyme 1" which plays a role in the regulation of polyamine synthesis by binding to and inhibiting ornithine decarboxylase. The expression levels OAZ1 gene can be determined by the levels of the corresponding mRNA or by the levels of the polypeptide encoded by said gene. In a preferred embodiment, the expression levels of the OAZ1 gene is determined by determining the expression levels of one or more of the transcripts defined under the accession numbers ENST00000602676.6, ENST00000582888.8, ENST00000588673.3, ENST00000590943.6, ENST00000586054.2, ENST00000593012.1, ENST00000581150.6, ENST00000592787.2, ENST00000592727.3, ENST00000589361.2 and ENST00000589739.3 in the Ensemble database under accession number ENSG00000104904 (Release 110).. In a preferred embodiment, the expression levels of the OAZ1 gene are determined by measuring the expression of the polypeptide defined under accession number P54368 of the Uniprot database, entry version 28-Jun-2023
The term "UBB" refers to the gene which encodes for the protein "Polyubiquitin-B" which exists either covalently attached to another protein, or free (unanchored). The term "MRPL19" refers to the gene which encodes for the protein "39S ribosomal protein L19, mitochondrial" which help in protein synthesis within the mitochondrion. The expression levels MRPL19gene can be determined by the levels of the corresponding mRNA or by the levels of the polypeptide encoded by said gene. In a preferred embodiment, the expression levels of the MRPL19gene is determined by determining the expression levels of one or more of the transcripts defined under the accession numbers ENST00000393909.7, ENST00000409374.5, ENST00000476622.1, ENST00000358788.10, ENST00000453233.1, ENST00000492255.1 and ENST00000493686.1 in the Ensemble database under accession number ENSG00000115364 (Release 110).. In a preferred embodiment, the expression levels of the MRPL19 gene are determined by measuring the expression of the polypeptide defined under accession number of the P49406 Uniprot database, entry version 28-Jun-2023
Once the expression levels of the genes or of the circRNA in relation to reference values for said genes or circRNAs have been determined and optionally normalized with the expression levels of the adequate housekeeping genes, it is necessary to identify if there are alterations in the expression of said genes (increase or decrease of the expression). The increase or decrease of the expression levels can be described both in increase/decrease percentage as well as fold increase/decrease. The expression level of a gene, either measured as the levels of the mRNA encoded by the genes or measured as the level of the polypeptide encoded by the gene is considered increased in a sample of the patient under study when the levels increase with respect to the reference values by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more. Likewise, the expression of a gene is considered increased in a sample of the patient under study when the levels increase with respect to the reference values by at least 1.1 fold, by at least 1.2 fold, by at least 1.3 fold, by at least 1.4 fold, by at least 1.5 fold, by at least 1.6 fold, by at least 1.7 fold, by at least 1.8 fold, by at least 1.9 fold, by at least 2 fold, by at least 3 fold, by at least 4 fold, by at least 5 fold, by at least 6 fold, by at least 7 fold, by at least 8 fold, by at least 9 fold, by at least 10 fold or more. Similarly, the expression of a gene is considered decreased when its levels decrease with respect to the reference sample by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100% (i.e., absent). Likewise, the expression of a gene is considered decreased in a sample of the patient under study when the levels decrease with respect to the reference values by at least 1.1 fold, by at least 1.2 fold, by at least 1.3 fold, by at least 1.4 fold, by at least 1.5 fold, by at least 1.6 fold, by at least 1.7 fold, by at least 1.8 fold, by at least 1.9 fold, by at least 2 fold, by at least 3 fold, by at least 4 fold, by at least 5 fold, by at least 6 fold, by at least 7 fold, by at least 8 fold, by at least 9 fold, by at least 10 fold or more.

In a particular embodiment of diagnostic method of the invention the level of MS4A1 is decreased by at least 1.1 fold, by at least 1.2 fold, by at least 1.3 fold, by at least 1.4 fold, by at least 1.5 fold, by at least 1.6 fold, by at least 1.7 fold, by at least 1.8 fold, by at least 1.9 fold, by at least 2 fold with respect to the reference value, preferably at least 1.5 fold with respect to the reference value.

In a particular embodiment of diagnostic method of the invention the level of hsa_circ_0000994 is decreased by at least 1.1 fold, by at least 1.2 fold, by at least 1.3 fold, by at least 1.4 fold, by at least 1.5 fold, by at least 1.6 fold, by at least 1.7 fold, by at least 1.8 fold, by at least 1.9 fold, by at least 2 fold with respect to the reference value, preferably at least 1.5 fold with respect to the reference value.

In a particular embodiment of diagnostic method of the invention the level of hsa_circ_0012673 is decreased by at least 1.1 fold, by at least 1.2 fold, by at least 1.3 fold, by at least 1.4 fold, by at least 1.5 fold, by at least 1.6 fold, by at least 1.7 fold, by at least 1.8 fold, by at least 1.9 fold, by at least 2 fold with respect to the reference value, preferably at least 1.5 fold with respect to the reference value.

In another particular embodiment of method I of the invention the level of DDB2 is decreased by at least 1.1 fold, by at least 1.2 fold, by at least 1.3 fold, by at least 1.4 fold, by at least 1.5 fold, by at least 1.6 fold, by at least 1.7 fold, by at least 1.8 fold, by at least 1.9 fold, by at least 2 fold with respect to the reference value, preferably at least 1.5 fold with respect to the reference value.

Once the expression levels of the gene or genes or the levels of the circRNA as defined in the present invention are determined, the lung nodules in a patient are characterized as being benign or cancerous.

The expression "is indicative that the lung nodules are cancerous nodules" as it will be understoode expert in the field will understand, although preferred to be, need not be correct for 100% of the patients to be diagnosed or evaluated. The term, however, requires that a statistically significant portion of subjects can be identified as having an increased probability of having a given outcome. Whether a subject is statistically significant can be determined without further ado by the person skilled in the art using various well known statistic evaluation tools, e.g., determination of confidence intervals, p-value determination, cross-validated classification rates and the like etc. Details are found in Dowdy and Wearden, Statistics for Research, John Wiley & Sons, New York 1983. Preferred confidence intervals are at least 50%, at least 60%, at least 70%, at least 80%, at least 90% or at least 95%. The p-values are, preferably, 0.05, 0.01, 0,005 or lower.

In an additional embodiment, the determination of whether the lung nodules in the patient are benign or cancerous is further assisted by an analysis of a biopsy sample from one or more nodules obtained from the patient.

In some embodiments, when lung cancer is ruled out, or a nodule is prognosed to be benign, the subject is monitored periodically. In some embodiments, the periodic monitoring is a pulmonary function test (PET), pulmonary imaging, a biopsy or any combination thereof. In some embodiments, said pulmonary imaging is an x-ray, a chest computed tomography (CT) scan, or a positron emission tomography (PET) scan. In some embodiments, said pulmonary nodule has a diameter of less than or equal to 3 cm. In some embodiments, said pulmonary nodule has a diameter of about 0.8 cm up to 3.0 cm.

In some embodiments, when the lung nodules in the patient are characterized as being cancerous, the subject is monitored periodically. In some embodiments, the subject has low to moderate cancer risk.

### Method for selecting a subject for a lung cancer therapy

Another aspect of the present invention relates to a method for selecting a patient having one or more lung nodules for lung cancer therapy (hereinafter "method for personalized therapy of the invention), the method comprising:
(i) determining the expression level of the MS4A1 gene in a sample from the subject, and
(ii) comparing the level obtained in (i) to a reference value,
wherein an expression level of MS4A1 that is decreased with respect to the reference value is indicative that the subject is selected for a lung cancer therapy.

A further aspect of the present invention related to a method for selecting a subject having one or more lung nodules for lung cancer therapy comprising:
(i) determining the level of the hsa_circ_0000994 and/or hsa_circ_0012673 circular RNAs in a sample from the patient, and
(ii) comparing the level obtained in (i) to a reference value,
wherein an increased level of the hsa_circ_0000994 circular RNA and/or a decreased level of the hsa_circ_0012673 circular RNA with respect to their respective reference values is indicative that the subject is selected for lung cancer therapy.

From here onwards both methods are referred to as "therapy assignation method" of the invention.

All previous embodiment and definitions are equally and validly applicable to the present aspect.

In one embodiment, the therapy assignation method of the invention based on the determination of the expression levels of the MS4A1 gene further comprises determining the expression level of the DDB2 gene wherein an expression level of the DDB2 gene that is increased with respect to the reference value is indicative that the subject is selected for lung cancer therapy.

In another embodiment, the therapy assignation method of the invention based on the determination of the expression levels of the MS4A1 gene and of the DDB2 gene further comprise the determination of the expression levels of one or more of the SPIB, TAPBPL, TLR8, LY9, RELA, CXCL8, CD74, KLRK1, CD3D and DUSP1 genes in the sample from the subject, wherein if the expression levels of SPIB, TAPBPL, TLR8, LY9, RELA, CXCL8, CD74, KLRK1, CD3D and DUSP1 genes are decreased with respect to the reference value, then it is indicative that the subject is selected for lung cancer therapy, then the subject is selected for lung cancer therapy.

In some embodiments, the therapy assignation method of the invention, the lung cancer therapy is selected from surgical resection of the lung nodules, chemotherapy, radiation therapy, targeted therapy or a combination thereof.

In some embodiments, the therapy assignation method of the invention, the sample in which the determination of the expression levels of one or more genes or the determination of the levels of the different circRNA is selected from blood, plasma or serum. In a yet more preferred embodiment, the sample is plasma.

In some embodiments of the therapy assignation method of the invention, the determination of the expression levels of the one or more genes is carried out by determining the levels of RNA transcripts of the gene(s). In yet another embodiment, the determination of the levels of the RNA transcripts is measured in the circulating free RNA.

In some embodiments of the therapy assignation method of the invention, the expression levels of the one or more genes is normalized using the expression levels of one or more housekeeping genes. In some embodiments, wherein the method for personalized therapy of the invention is based on the determination of the expression levels of one or genes including the MS4A1 gene, then the housekeeping gene is selected from a group consisting of the OAZ1, UBB, MRPL19 genes. In some embodiments, wherein the method for personalized therapy of the invention is based on the determination of the levels of one or circRNAs including the hsa_circ_0000994 and/or hsa_circ_0012673, then the housekeeping gene is selected from a group consisting of the UBB, the MRPL19 gene or a combination thereof.

In some embodiments, the reference value that is used for the determination of whether the expression levels of one or more genes or the levels of one or more circRNA are the expression levels of said one or more genes or the levels of said one or more circRNA present in a patient with benign lung nodules. It will be understood that the reference value is typically determined in the same type of sample in which the expression level/level is determined. In an embodiment, wherein the expression levels of the one or more genes or the levels of the one or more circRNA are determined in a biofluid from the patient, then the reference value will be obtained also from a biofluid, preferably in the same type of biofluid.

Once the patient has been characterized using the methods of the present invention, the patient will be selected for a therapy for lung cancer if the lung nodules are characterized as being cancerous.

(As used herein, the terms "therapy", "treat", "treatment", "treatment", or "amelioration" are used interchangeably. The term refers to therapeutic treatment, the purpose of which is to reverse, reduce, suppress, delay or stop the progression or severity of the condition associated with the disease or disorder. The term "treatment" includes reducing or alleviating at least one adverse effect or condition of a condition, such as cancer, a disease or disorder. Treatment is usually "effective" when one or more symptoms or clinical markers are reduced. Alternatively, treatment is "effective" if disease progression is delayed or halted. That is, "treatment" includes not only the improvement of symptoms or markers, but also the interruption of at least a condition that indicates the progression or worsening of symptoms that would be expected in the absence of treatment. The beneficial or desirable clinical outcome, whether detectable or not, is a reduction in one or more symptoms, a reduction in the extent of the disease, a stable (ie, not aggravated) condition of the disease, a disease These include, but are not limited to, delayed or slowed progression, amelioration or alleviation of the disease state, and remission (partial or total). The term "treatment" of a disease also includes providing relief from symptoms or side effects of the disease (including symptomatic treatment). In the present context the term therapy refers to lung cancer therapy. In a particular embodiment of the present invention the lung cancer therapy is selected from surgery, chemotherapy, radiation therapy, targeted therapy or a combination thereof. In another preferred embodiment the lung cancer therapy is surgical resection.

The term "surgery", as the expert in the field is aware, refers to invasive measures for treating the cancer, in particular, the excision, resection or removal of the tumor tissue.

The term "chemotherapy" refers to the use of drugs to treat cancer. The drugs are generally administered through oral or intravenous route. Sometimes, chemotherapy is used together with radiation treatment. A "chemotherapeutic drug" is used to connote a compound or composition that is administered in the treatment of cancer. These agents or drugs are categorized by their mode of activity within a cell, for example, whether and at what stage they affect the cell cycle. Alternatively, an agent may be characterized based on its ability to directly cross-link DNA, to intercalate into DNA, or to induce chromosomal and mitotic aberrations by affecting nucleic acid synthesis. Examples of chemotherapy drugs, without limitation, which can be used in the treatment of lung cancer are Abraxane (Paclitaxel Albumin-stabilized Nanoparticle Formulation), Adagrasib, Afatinib Dimaleate, Afinitor (Everolimus), Afinitor Disperz (Everolimus), Alecensa (Alectinib), Alectinib, Alimta (Pemetrexed Disodium), Alunbrig (Brigatinib), Alymsys (Bevacizumab), Amivantamab-vmjw, Atezolizumab, Avastin (Bevacizumab), Bevacizumab, Brigatinib, Capmatinib Hydrochloride, Cemiplimab-rwlc, Ceritinib, Crizotinib, Cyramza (Ramucirumab), Dabrafenib Mesylate, Dacomitinib, Docetaxel, Doxorubicin Hydrochloride, Durvalumab, Enhertu (Fam-Trastuzumab Deruxtecan-nxki), Entrectinib, Erlotinib Hydrochloride, Everolimus, Exkivity (Mobocertinib Succinate), Fam-Trastuzumab Deruxtecan-nxki, Gavreto (Pralsetinib), Gefitinib, Gilotrif (Afatinib Dimaleate), Gemcitabine Hydrochloride, Gemzar (Gemcitabine Hydrochloride), Imfinzi (Durvalumab), Imjudo (Tremelimumab-actl), Infugem (Gemcitabine Hydrochloride), Ipilimumab, Iressa (Gefitinib), Keytruda (Pembrolizumab), Krazati (Adagrasib), Libtayo (Cemiplimab-rwlc), Lorbrena (Lorlatinib), Lorlatinib, Lumakras (Sotorasib), Mekinist (Trametinib Dimethyl Sulfoxide), Methotrexate Sodium, Mobocertinib Succinate, Mvasi (Bevacizumab), Necitumumab, Nivolumab, Opdivo (Nivolumab), Osimertinib Mesylate, Paclitaxel, Paclitaxel Albumin-stabilized Nanoparticle Formulation, Pembrolizumab, Pemetrexed Disodium, Portrazza (Necitumumab), Pralsetinib, Ramucirumab, Retevmo (Selpercatinib), Rozlytrek (Entrectinib), Rybrevant (Amivantamab-vmjw), Selpercatinib, Sotorasib, Tabrecta (Capmatinib Hydrochloride), Tafinlar (Dabrafenib Mesylate), Tagrisso (Osimertinib Mesylate), Tarceva (Erlotinib Hydrochloride), Taxotere (Docetaxel), Tecentriq (Atezolizumab), Tepmetko (Tepotinib Hydrochloride), Tepotinib Hydrochloride, Trametinib Dimethyl Sulfoxide, Tremelimumab-actl, Trexall (Methotrexate Sodium), Vizimpro (Dacomitinib), Vinorelbine Tartrate, Xalkori (Crizotinib), Yervoy (Ipilimumab), Zirabev (Bevacizumab), Zykadia (Ceritinib). Chemotherapy may be administered with using a single drug or a combination of two drugs which are complementary in their action mode of treatment.

Chemotherapy may be administered before, "neoadjuvant" or after, "adjuvant", another treatment such as surgical resection of the tumor. The terms "neoadjuvant chemotherapy" and "adjuvant chemotherapy", at used herein refers to a treatment of cancer with standard chemotherapeutic agents before and after, respectively, surgery where all detectable disease has been removed, to either reduce the size of the tumor and/or reduce the risk of small amounts of cancer remaining.

The term "radiation therapy" may be used interchangeably with the term "radiotherapy", is a type of cancer treatment that uses beams of intense energy to kill cancer cells. Radiation therapy most often uses X-rays, but gamma rays, electron beams, or protons also can be used. The term "radiation therapy" most often refers to external beam radiation therapy. During this type of radiation, the high-energy beams come from a machine outside of the patient's body that aims the beams at a precise point on the body.

The term "targeted therapy", as used herein, relates to application to a patient of a chemical substance known to block growth of cancer cells by interfering with specific molecules known to be necessary for tumorigenesis or cancer or cancer cell growth. Examples known to the skilled artisan are small molecules like, e.g. PARP-inhibitors (e.g. Iniparib), or monoclonal antibodies like, e.g., Trastuzumab. An example of targeted therapy is immunotherapy. The term "immunotherapy" as used herein relates to the treatment of cancer by modulation of the immune response of a subject, e.g. as cell based immunotherapy. Said modulation may be inducing, enhancing, or suppressing said immune response. The term "cell based immunotherapy" relates to a cancer therapy comprising application of immune cells, e.g. T-cells, preferably tumor-specific NK cells, to a subject.

### Method for selecting a subject for lung cancer therapy and treating the subject

Another aspect of the present invention relates to a method for selecting a subject having one or more lung nodules for lung cancer therapy and treating the subject with said therapy comprising:
(i) determining the expression level of MS4A1 in a sample from the subject, and
(ii) comparing the level obtained in (i) to a reference value,
(iii) treating the subject with the therapy if the subject is identified as having decreased expression level of MS4A1 gene with respect to the reference value.

A further aspect of the present invention relates to a method for selecting a subject having one or more lung nodules for lung cancer therapy and treating the subject with said therapy comprising:
(a) determining the level of the hsa_circ_0000994 and/or hsa_circ_0012673 circular RNAs in a sample from the patient, and
(ii) comparing the level obtained in (i) to a reference value,
(iii) treating the subject with the therapy if the patient is identified as having increased levels of the hsa_circ_0000994 circular RNA and/or a decreased level of the hsa_circ_0012673 circular RNA with respect to their respective reference values.

These two methods will be referred below collectively as "method for personalized therapy of the invention". All previous embodiment and definitions are equally and validly applicable to the present aspect.

In one embodiment, the method for personalized therapy of the invention based on the determination of the expression levels of the MS4A1 gene further comprises determining the expression level of the DDB2 gene wherein an expression level of the DDB2 gene that is increased with respect to the reference value is indicative that the subject is selected for lung cancer therapy.

In another embodiment, the method for personalized therapy of the invention based on the determination of the expression levels of the MS4A1 gene and of the DDB2 gene further comprise the determination of the expression levels of one or more of the SPIB, TAPBPL, TLR8, LY9, RELA, CXCL8, CD74, KLRK1, CD3D and DUSP1 genes in the sample from the subject, wherein if the expression levels of SPIB, TAPBPL, TLR8, LY9, RELA, CXCL8, CD74, KLRK1, CD3D and DUSP1 genes are decreased with respect to the reference value, then it is indicative that the subject is selected for lung cancer therapy, then the subject is selected for lung cancer therapy.

In some embodiments of the methods for personalized therapy of the invention, the lung cancer therapy is selected from surgical resection of the lung nodules, chemotherapy, radiation therapy, targeted therapy or a combination thereof. These terms have been described above in the context of the therapy assignation method and are equally applicable to the present methods.

In some embodiments, the methods for personalized therapy of the invention, the sample in which the determination of the expression levels of one or more genes or the determination of the levels of the different circRNA is selected from blood, plasma or serum. In a yet more preferred embodiment, the sample is plasma.

In some embodiments of the methods for personalized therapy of the invention, the determination of the expression levels of the one or more genes is carried out by determining the levels of RNA transcripts of the gene(s). In yet another embodiment, the determination of the levels of the RNA transcripts is measured in the circulating free RNA.

In some embodiments of the methods for personalized therapy of the invention, the expression levels of the one or more genes is normalized using the expression levels of one or more housekeeping genes. In some embodiments, wherein the method for personalized therapy of the invention is based on the determination of the expression levels of one or genes including the MS4A1 gene, then the housekeeping gene is selected from a group consisting of the OAZ1, UBB, MRPL19 genes. In some embodiments, wherein the method for personalized therapy of the invention is based on the determination of the levels of one or circRNAs including the hsa_circ_0000994 and/or hsa_circ_0012673, then the housekeeping gene is selected from a group consisting of the UBB, the MRPL19 gene or a combination thereof.

In some embodiments, the reference value that is used for the determination of whether the expression levels of one or more genes or the levels of one or more circRNA are the expression levels of said one or more genes or the levels of said one or more circRNA present in a patient with benign lung nodules. It will be understood that the reference value is typically determined in the same type of sample in which the expression level/level is determined. In an embodiment, wherein the expression levels of the one or more genes or the levels of the one or more circRNA are determined in a biofluid from the patient, then the reference value will be obtained also from a biofluid, preferably in the same type of biofluid.

Once the patient has been characterized using the methods of the present invention, the patient will be selected for a therapy for lung cancer if the lung nodules are characterized as being cancerous.

All previous embodiment and definitions are equally and validly applicable to the present aspect.

### Kit of the invention

In another aspect of the present invention relates to a kit comprising specific reagents for carrying out the methods according to the invention, including the diagnostic methods based on the determination of the expression levels of one or more genes and on the determination of the levels of circRNA, the therapy assignation methods based in the determination of the expression levels of certain genes and on the determination of the levels of circRNA as well as the methods for personalized therapy based on the determination of the expression levels of one or more genes and on the determination of the levels of circRNA

All previous embodiment and definitions are equally and validly applicable to the present aspect.

In the context of the present invention, "kit" is understood as a product containing the different reagents necessary for carrying out the methods of the invention packed so as to allow their transport and storage. Additionally, the kits of the invention can contain instructions for the simultaneous, sequential or separate use of the different components which are in the kit. Said instructions can be in the form of printed material or in the form of an electronic support capable of storing instructions such that they can be read by a subject, such as electronic storage media (magnetic disks, tapes and the like), optical media (CD-ROM, DVD) and the like.

In a particular embodiment the kit of the invention the specific reagents are adequate for determining the expression levels of the genes MS4A1 and DDB2, SPIB, TAPBPL, TLR8, LY9, RELA, CXCL8, CD74, KLRK1, CD3D and/or DUSP1.

In a particular embodiment the kit of the invention the specific reagents are adequate for determining the levels of the circular RNAs hsa_circ_0000994 and/or hsa_circ_0012673.

In a further particular embodiment the kit of the invention further comprises reagents adequate for determining the expression levels of one or more housekeeping genes, preferably one or more of the housekeeping genes OAZ1, UBB and MRPL19.

The expression "reagent which allows determining the expression level of a gene" means a compound or set of compounds that allows determining the expression level of a gene both by means of the determination of the level of mRNA or by means of the determination of the level of protein. Thus, reagents of the first type include probes capable of specifically hybridizing with the mRNAs encoded by said genes. Reagents of the second type include compounds that bind specifically with the proteins encoded by the marker genes and preferably include antibodies, although they can be specific aptamers.

In further embodiments of the kit of the invention, the reagents adequate for the determination of the expression levels of one or more genes or circRNAs comprise at least 55% at least 60%, at least 65%, at least 70%, at least 75%, at least 80%, at least 90%, at least 95%, at least 96%, at least 97%, at least 98% or at least 99% of the total amount of reagents forming the kit.

In a particular embodiment of the kit of the invention, the reagents of the kit are nucleic acids which are capable of specifically detecting the RNA level of the genes or circRNAs mentioned above and/or the level of proteins encoded by one or more of the genes mentioned above. Nucleic acids capable of specifically hybridizing with the genes mentioned above can be one or more pairs of primer oligonucleotides for the specific amplification of fragments of the mRNAs (or of their corresponding cDNAs) of said genes or circRNAs.

In a preferred embodiment, the first component of the kit of the invention comprises a probe which can specifically hybridize to the genes mentioned above.

The term "specifically hybridizing", as used herein, refers to conditions which allow hybridizing of two polynucleotides under high stringent conditions or moderately stringent conditions.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so.

In the event that the expression levels of several of the genes identified in the present invention are to be simultaneously determined, it is useful to include probes for all the genes the expression of which is to be determined in a microarray hybridization.

The microarrays comprise a plurality of nucleic acids that are spatially distributed and stably associated to a support (for example, a biochip). The nucleic acids have a sequence complementary to particular subsequences of genes the expression of which is to be detected, therefore are capable of hybridizing with said nucleic acids. In the methods of the invention, a microarray comprising an array of nucleic acids is put into contact with a preparation of nucleic acids isolated from the patient object of the study. The incubation of the microarray with the preparation of nucleic acids is carried out in conditions suitable for the hybridization. Subsequently, after the elimination of the nucleic acids which have not been retained in the support, the hybridization pattern is detected, which provides information on the genetic profile of the sample analyzed. Although the microarrays are capable of providing both qualitative and quantitative information of the nucleic acids present in a sample, the invention requires the use of arrays and methodologies capable of providing quantitative information.

The invention contemplates a variety of arrays with regard to the type of probes and with regard to the type of support used. The probes included in the arrays that are capable of hybridizing with the nucleic acids can be nucleic acids or analogs thereof which maintain the hybridization capacity such as for example, nucleic acids in which the phosphodiester bond has been substituted with a phosphorothioate, methylimine, methylphosphonate, phosphoramidate, guanidine bond and the like, nucleic acids in which the ribose of the nucleotides is substituted with another hexose, peptide nucleic acids (PNA). The length of the probes can of 5 to 50 nucleotides and, preferably, of 7, 10, 15, 20, 25, 30, 35, 40, 45, 50, 55, 60, 65, 70, 75, 100 nucleotides and vary in the range of 10 to 1000 nucleotides, preferably in the range of 15 to 150 nucleotides, more preferably in the range of 15 to 100 nucleotides and can be single-stranded or doublestranded nucleic acids. The array can contain all the specific probes of a certain mRNA of a certain length or can contain probes selected from different regions of an mRNA.

The microarrays of the invention contain not only specific probes for the polynucleotides indicating a determined pathophysiological situation, but also containing a series of control probes, which can be of three types: normalization controls, expression level controls and hybridization controls.

In the event that the expression levels of the genes according to the present invention is determined by measuring the levels of the polypeptide or polypeptides encoded by said gene or genes, the kits according to the present invention comprise reagents which are capable of specifically binding to said polypeptides.

For this purpose, the arrays of antibodies are useful. The antibodies of the array include any immunological agent capable of binding to a ligand with high affinity, including IgG, IgM, IgA, IgD and IgE, as well as molecules similar to antibodies which have an antigen binding site, such as Fab', Fab, F(ab')2, single domain antibodies or DABS, Fv, scFv and the like.

### Computer system

In a particular embodiment of any of the methods of the invention, the method is computer-implemented.

The term "computer implemented" as used herein refers to fact that the steps of the methods according to the invention are performed by a computer or a computer system. The term "computer implemented" is meant to also include the term "partially computer-implemented", which refers to a method in which only particular steps, e.g., calculating steps, are computer-implemented, whereas other steps of the method are not.

A further aspect of the present invention relates to a computer system comprising one or more programs, wherein the one or more programs are include instructions for performing any of the methods of the invention.

Another aspect of the present invention relates to a computer-readable storage medium storing one or more programs, the one or more programs comprising instructions, which when executed by one or more processors of an electronic device, cause the electronic device to perform any of the methods of the invention.

As will be appreciated by one of skill in the art, the computer readable instructions cause the computing system to carry out the method according to the invention. The device may take the form of an entire hardware embodiment or an embodiment combining software and hardware aspects. Furthermore, the present invention may include a computer program product on a computer-usable storage medium having computer-usable program code means embodied in the medium. Any suitable computer readable medium may be utilized including hard disks, CD-ROMs, optical storage devices, or magnetic storage devices. The computer-usable or computer-readable medium may be or include, for example, but not limited to, an electronic, magnetic, optical, electromagnetic, infrared, or semiconductor system, apparatus, device, or propagation medium. More specific examples (a non-exhaustive list) of the computer readable medium would include the following: an electrical connection having one or more wires, a portable computer diskette, a random access memory (RAM), a read-only memory (ROM), an erasable programmable read-only memory (EPROM or Flash memory), an optical fiber, and a portable compact disc read-only memory (CD-ROM), a CD ROM, a DVD (digital video disk), or other electronic storage medium, even when accessed through wireless connection. Note that the computer-usable or computer-readable medium could even be paper or another. Suitable medium upon which the program is printed, as the program can be electronically captured, via, for instance, optical scanning of the paper or other medium, then compiled, interpreted or otherwise processed in a suitable manner if necessary, and then stored in a computer memory.

Computer program code for carrying out operations of the present invention may be written in an object-oriented programming language such as R, Python, Matlab, Java, and Javascript, C #, smalltalk or C++. However, the computer program code for carrying out operations of the present invention may also be written in conventional procedural programming languages, such as the "C" or FORTRAN programming language or even assembly language. The program code may execute entirely on the user's computer, partly on the user's computer, as a stand-alone software package, partly on the user's computer and partly on a remote computer or entirely on the remote computer. In the latter scenario, the remote computer may be connected to the user's computer through a local area network (LAN) or a wide area network (WAN), or the connection may be made to an external computer (for example, through the Internet using an Internet Service Provider).

All previous embodiment and definitions are equally and validly applicable to the present aspect.

The invention is defined by the following aspects:
1. A method for determining whether lung nodules in a patient are benign or cancerous comprising:
   (i) determining the expression level of the MS4A1 gene in a sample from the patient, and
   (ii) comparing the level obtained in (i) to a reference value,
      wherein an expression level of the MS4A1 gene that is decreased with respect to the reference value is indicative that the lung nodules are cancerous nodules.
2. A method for selecting a subject having one or more lung nodules for a lung cancer therapy comprising:
   (i) determining the expression level of the MS4A1 gene in a sample from the subject, and
   (ii) comparing the level obtained in (i) to a reference value,
      wherein an expression level of the MS4A1 gene that is decreased with respect to the reference value is indicative that the subject is selected for a lung cancer therapy.
3. A method for selecting a subject having one or more lung nodules for lung cancer therapy and treating the subject with said therapy comprising:
   (i) determining the expression level of the MS4A1 gene in a sample from the subject, and
   (ii) comparing the level obtained in (i) to a reference value,
   (iii) treating the subject with the therapy if the subject is identified as having decreased expression level of the MS4A1 gene with respect to the reference value.
4. The method according to any of aspects 1 to 3 wherein step (i) further comprises determining the expression level of the DDB2 gene in the sample from the subject wherein,
   - an expression level of the DDB2 gene that is increased with respect to the reference value is indicative that the lung nodules are cancerous nodules,
   - an expression level of the DDB2 gene that is increased with respect to the reference value is indicative that the subject is selected for lung cancer therapy or
   - the subject is treated with the therapy if the subject is identified as having increased expression level of the DDB2 gene with respect to the reference value
5. The method according to aspect 4 wherein step (i) further comprises determining the expression level of one or more of the SPIB, TAPBPL, TLR8, LY9, RELA, CXCL8, CD74, KLRK1, CD3D and DUSP1 genes in the sample from the subject, wherein
   - if the expression levels of the SPIB, TAPBPL, TLR8, LY9, RELA, CXCL8, CD74, KLRK1, CD3D and DUSP1 genes are decreased with respect to the reference value, then it is indicative that the lung nodules are cancerous nodules,
   - if the expression levels of the SPIB, TAPBPL, TLR8, LY9, RELA, CXCL8, CD74, KLRK1, CD3D and DUSP1 genes are decreased with respect to the reference value, then it is indicative that the subject is selected for lung cancer therapy.
   - the subject is treated with the therapy if the subject is identified as having decreased levels of the SPIB, TAPBPL, TLR8, LY9, RELA, CXCL8, CD74, KLRK1, CD3D and DUSP1 genes.
6. The method according to any of aspects 1 to 5 wherein step (i) comprises determining the expression level of the MS4A1, DDB2, SPIB, TAPBPL, TLR8, LY9, RELA, CXCL8, CD74, KLRK1, CD3D and DUSP1 genes in the sample from the subject, wherein
   - an increased expression level of the DDB2 with respect to the reference value and decreased expression levels of the MS4A1, DDB2, SPIB, TAPBPL, TLR8, LY9, RELA, CXCL8, CD74, KLRK1, CD3D and DUSP1 genes with respect to their respective reference values are indicative that the lung nodules are cancerous nodules,
   - an increased expression level of the DDB2 with respect to the reference value and decreased expression levels of the MS4A1, DDB2, SPIB, TAPBPL, TLR8, LY9, RELA, CXCL8, CD74, KLRK1, CD3D and DUSP1 genes with respect to their respective reference values are indicative that the subject is selected for lung cancer therapy.
   - the subject is treated with the therapy if the subject is identified as having increased expression level of the DDB2 with respect to the reference value and decreased expression levels of the MS4A1, DDB2, SPIB, TAPBPL, TLR8, LY9, RELA, CXCL8, CD74, KLRK1, CD3D and DUSP1 genes.
7. The method according to any of aspects 1 to 6 wherein the expression levels are normalized based on the expression level of at least one housekeeping gene.
8. The method according to aspect 7 wherein the housekeeping gene is selected from a group consisting of the OAZ1, UBB, MRPL19 genes or a combination thereof.
9. A method for determining whether lung nodules in a patient are benign or are cancerous comprising:
   (i) determining the level of the hsa_circ_0000994 and/or hsa_circ_0012673 circular RNAs in a sample from the patient, and
   (ii) comparing the level obtained in (i) to a reference value,
      wherein an increased level of the hsa_circ_0000994 circular RNA and/or a decreased level of the hsa_circ_0012673 circular RNA with respect to their respective reference values is indicative that the lung nodules are cancerous nodules.
10. A method for selecting a subject having one or more lung nodules for lung cancer therapy comprising:
   (i) determining the level of the hsa_circ_0000994 and/or hsa_circ_0012673 circular RNAs in a sample from the patient, and
   (ii) comparing the level obtained in (i) to a reference value,
      wherein an increased level of the hsa_circ_0000994 circular RNA and/or a decreased level of the hsa_circ_0012673 circular RNA with respect to their respective reference values is indicative that the subject is selected for lung cancer therapy.
11. A method for selecting a subject having one or more lung nodules for lung cancer therapy and treating the subject with said therapy comprising:
   (i) determining the level of the hsa_circ_0000994 and/or hsa_circ_0012673 circular RNAs in a sample from the patient, and
   (ii) comparing the level obtained in (i) to a reference value,
   (iii) treating the subject with the therapy if the patient is identified as having increased levels of the hsa_circ_0000994 circular RNA and/or a decreased level of the hsa_circ_0012673 circular RNA with respect to their respective reference values.
12. The method according to any of aspects 9 to 11 wherein the expression levels is normalized based on the expression level of at least one housekeeping gene.
13. The method according to aspect 12 wherein the housekeeping gene is selected from a group consisting of the UBB, the MRPL19 gene or a combination thereof.
14. The method according to any of aspects 1 to 13 wherein
   (i) The determining of whether the lung nodules in the patient are benign or cancerous,
   (ii) The selection of the subject having one or more lung nodules for a lung cancer therapy or
   (iii) The selecting of the subject for lung cancer therapy and the treatment of the subject:
      is further assisted by an analysis of a biopsy sample from one or more nodules obtained from the patient.
15. The method according to aspect 2, 3, 1 or 11 wherein the lung cancer therapy is selected from surgical resection of the lung nodules, chemotherapy, radiation therapy, targeted therapy or a combination thereof.
16. The method according to any of aspects 1 to 15 wherein the lung cancer is non-small cell lung cancer (NSCLC).
17. The method according to any of aspects 1 to 16 wherein the sample is selected from blood, plasma or serum.
18. The method according to aspect 17 wherein the sample is plasma.
19. The method according to any of aspects 1 to 18 wherein the determination of the expression level of the one or more genes is carried out by determining the levels of RNA transcripts of the gene(s).
20. The method according to aspect 19 wherein the determination of the levels of the RNA transcripts is measured in the circulating free RNA.
21. The method according to any of aspects 1 to 20 wherein the reference value relates to the expression level of the gene in a patient with benign lung nodules.
22. A kit comprising specific reagents for carrying out the method according to any of aspects 1 to 21.
23. A computer system comprising one or more programs, wherein the one or more programs are include instructions for performing the method of any one of aspects 1 to 21.
24. A computer-readable storage medium storing one or more programs, the one or more programs comprising instructions, which when executed by one or more processors of an electronic device, cause the electronic device to perform the method of any one of aspects 1- 21.

The invention will be described by way of the following examples which are to be considered as merely illustrative and not limitative of the scope of the invention.

### EXAMPLES

### METHODS

For each patient, 10 mL of peripheral blood was collected in one tube (with EDTA or another anticoagulant).

Circulating-free RNA (cfRNA) was isolated from the plasma of healthy donors (N=33), patients with non-tumor nodules (115) and patients with NSCLC confirmed from tumor nodules (N=149, using an automatic extraction method (Qiasymphony, Qiagen). Purified cfRNA was quantified by Nanodrop, retrotranscribed and pre-amplified (14 cycles) using a Low RNA Input Amplification kit (NanoString Technologies). Gene expression analysis was performed on the nCounter platform using the PanCancer IO360TM (NanoString Technologies), which can detect 770 transcripts related to tumor biology, tumor micro-environment and immune response.

### RESULTS

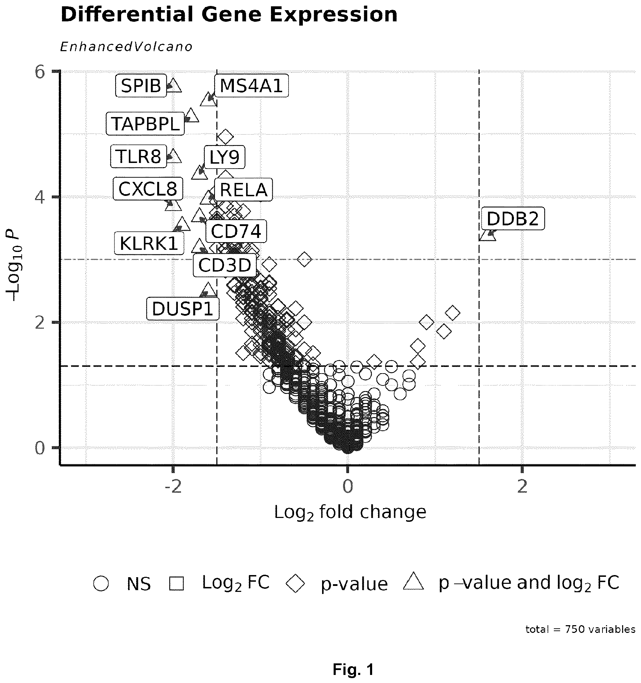

A bioinformatics analysis using machine learning normalized the data generated with NanoStringNorm R library. Log data with P<0.05 and fold change (FC) FC >1.5 as cutoff for cancer vs healthy and healthy donor samples. Wilcoxon-Mann-Whitney test was performed for the housekeeping genes between the groups using the data with three categories: Raw count, log of Raw count and normalized count). With the results of the three types of analysis three housekeeping genes were selected as more stables for both groups DNAJC14, G6PD and OAZ1 (Figure 1A-1C).

Next, differential expression analysis between cancer vs healthy and healthy donor samples was analyzed, and 11 genes were detected using as cutoff p.value 0.05 and FC of 1.5: TAPBPL, CXCL8, LY9, KLRK1, TLR8, CD3D, MS4A1, RELA, DDB2, SPIB and CD74 (Table1 and Figure 1).

**Table 1. Differential expression of the selected 11 genes using as cutoff p. value 0.05 and FC of 1.5**

| **Gene** | **Mean** | **SD** | **CV** | **Missing** | **P_Class1** | **FC_Class1** |
|---|---|---|---|---|---|---|
| **TAPBPL** | 4.3 | 3.4 | 79 | 36.3 | 7.10E-06 | -1.7 |
| **CXCL8** | 7.6 | 4.5 | 59.4 | 19.1 | 1.80E-04 | -1.9 |
| **LY9** | 5.1 | 3.7 | 72.3 | 30.7 | 6.80E-05 | -1.7 |
| **KLRK1** | 7 | 4.5 | 64 | 26.1 | 3.70E-04 | -1.8 |
| **TLR8** | 5.6 | 4.2 | 75.7 | 32 | 2.50E-05 | -2 |
| **CD3D** | 8.3 | 4.3 | 51.6 | 16.8 | 7.20E-04 | -1.7 |
| **MS4A1** | 9.4 | 3.1 | 32.6 | 6.9 | 6.10E-06 | -1.6 |
| **RELA** | 7.4 | 3.6 | 48.6 | 15.8 | 7.20E-05 | -1.6 |
| **DDB2** | 3.5 | 4 | 113.8 | 40.9 | 4.00E-04 | 1.6 |
| **SPIB** | 6.1 | 3.7 | 60.3 | 23.4 | 2.10E-06 | -2 |
| **CD74** | 5.8 | 4 | 68.5 | 27.4 | 2.70E-04 | -1.6 |

Finally, a feature selection by Random feature elimination using Bagged Tree algorithm was performed and the selection returns 12 features to get with an accuracy of 0.68 (Table2).

**Table 2: Feature selection by Random Feature Elimination. The best subset of genes by the feature selection is shown in italics.**

| **Gene Subset+** | **Delete highly correlated genes*** | **Gene Features** | **Differentially expressed genes** | **Accuracy** | **Kappa** |
|---|---|---|---|---|---|
| All | no | 750 | 11 | 0.646464 | 0.2929 |
| All | yes | 26 | 4 | 0.656565 | 0.3132 |
| DEpv | no | 15 | 4 | 0.673400 | 0.3467 |
| *DEpv* | *yes* | 12 | 2 | *0.676767* | *0.3536* |
| DEfc | no | 11 | 11 | 0.643097 | 0.2863 |
| DEfc | yes | 2 | 2 | 0.632996 | 0.2666 |
| DEpvfc | no | 11 | 11 | 0.643097 | 0.2863 |
| DEpvfc | yes | 2 | 2 | 0.632996 | 0.2666 |

| | | | | | |
|---|---|---|---|---|---|
| Note: + All 750 genes, DEpv : Differential expressed genes by p-value, DEfc: Differential expressed genes by fold change, DEpvfc: Differential expressed genes by p-value and fold change. P_class < 0.05 and \|FC_class\| >1.5 were used as a cutoff. * Spearman rank correlation, highly correlated > 0.75 | | | | | |

The features are *MS4A1, DDB2, SPIB, TAPBPL, TLR8, LY9, RELA, CXCL8,* CD74, *KLRK1, CD3D, DUSP1.* Two of the 12 features were also differentially expressed genes.

## Claims

1. A method for determining whether lung nodules in a patient are benign or cancerous comprising:
(i) determining the expression level of the MS4A1 gene in a sample from the patient, and
(ii) comparing the level obtained in (i) to a reference value,
wherein an expression level of the MS4A1 gene that is decreased with respect to the reference value is indicative that the lung nodules are cancerous nodules.

2. The method according to claim 1 wherein step (i) further comprises determining the expression level of the DDB2 gene in the sample from the subject wherein,
- an expression level of the DDB2 gene that is increased with respect to the reference value is indicative that the lung nodules are cancerous nodules,
- an expression level of the DDB2 gene that is increased with respect to the reference value is indicative that the subject is selected for lung cancer therapy or
- the subject is treated with the therapy if the subject is identified as having increased expression level of the DDB2 gene with respect to the reference value

3. The method according to claim 2 wherein step (i) further comprises determining the expression level of one or more of the SPIB, TAPBPL, TLR8, LY9, RELA, CXCL8, CD74, KLRK1, CD3D and DUSP1 genes in the sample from the subject, wherein
- if the expression levels of the SPIB, TAPBPL, TLR8, LY9, RELA, CXCL8, CD74, KLRK1, CD3D and DUSP1 genes are decreased with respect to the reference value, then it is indicative that the lung nodules are cancerous nodules,
- if the expression levels of the SPIB, TAPBPL, TLR8, LY9, RELA, CXCL8, CD74, KLRK1, CD3D and DUSP1 genes are decreased with respect to the reference value, then it is indicative that the subject is selected for lung cancer therapy.
- the subject is treated with the therapy if the subject is identified as having decreased levels of the SPIB, TAPBPL, TLR8, LY9, RELA, CXCL8, CD74, KLRK1, CD3D and DUSP1 genes.

4. The method according to any of claims 1 to 3 wherein step (i) comprises determining the expression level of the MS4A1, DDB2, SPIB, TAPBPL, TLR8, LY9, RELA, CXCL8, CD74, KLRK1, CD3D and DUSP1 genes in the sample from the subject, wherein
- an increased expression level of the DDB2 with respect to the reference value and decreased expression levels of the MS4A1, DDB2, SPIB, TAPBPL, TLR8, LY9, RELA, CXCL8, CD74, KLRK1, CD3D and DUSP1 genes with respect to their respective reference values are indicative that the lung nodules are cancerous nodules,
- an increased expression level of the DDB2 with respect to the reference value and decreased expression levels of the MS4A1, DDB2, SPIB, TAPBPL, TLR8, LY9, RELA, CXCL8, CD74, KLRK1, CD3D and DUSP1 genes with respect to their respective reference values are indicative that the subject is selected for lung cancer therapy.
- the subject is treated with the therapy if the subject is identified as having increased expression level of the DDB2 with respect to the reference value and decreased expression levels of the MS4A1, DDB2, SPIB, TAPBPL, TLR8, LY9, RELA, CXCL8, CD74, KLRK1, CD3D and DUSP1 genes.

5. The method according to any of claims 1 to 4 wherein the expression levels are normalized based on the expression level of at least one housekeeping gene.

6. The method according to claim 5 wherein the housekeeping gene is selected from a group consisting of the OAZ1, UBB, MRPL19 genes or a combination thereof.

7. A method for determining whether lung nodules in a patient are benign or are cancerous comprising:
(i) determining the level of the hsa_circ_0000994 and/or hsa_circ_0012673 circular RNAs in a sample from the patient, and
(ii) comparing the level obtained in (i) to a reference value,
wherein an increased level of the hsa_circ_0000994 circular RNA and/or a decreased level of the hsa_circ_0012673 circular RNA with respect to their respective reference values is indicative that the lung nodules are cancerous nodules.

8. The method according to claim 7 wherein the expression levels is normalized based on the expression level of at least one housekeeping gene.

9. The method according to claim 8 wherein the housekeeping gene is selected from a group consisting of the UBB, the MRPL19 gene or a combination thereof.

10. The method according to any of claims 1 to 9 wherein the lung cancer is non-small cell lung cancer (NSCLC).

11. The method according to any of claims 1 to 10 wherein the sample is selected from blood, plasma or serum.

12. The method according to claim 11 wherein the sample is plasma.

13. The method according to any of claims 1 to 12 wherein the determination of the expression level of the one or more genes is carried out by determining the levels of RNA transcripts of the gene(s).

14. The method according to claim 13 wherein the determination of the levels of the RNA transcripts is measured in the circulating free RNA.

15. The method according to any of claims 1 to 14 wherein the reference value relates to the expression level of the gene in a patient with benign lung nodules.
